# EUROPEAN PATENT APPLICATION

(11) **EP 4 331 589 A1**
(43) Date of publication of application: **06.03.2024**
(21) Application number: 22795862.6
(22) Date of filing: 27.04.2022
(51) Int. Cl.: A61K 31/655, A61K 8/49, A61P 17/00, A61Q 19/00

(54) **COMPOSITION FOR AMELIORATING ABNORMALITY OF SKIN TISSUE**

(30) Priority: 28.04.2021 JP 2021076617
(71) Applicant: Kyoto University, Kyoto-shi, Kyoto 606-8501 (JP)
(72) Inventor: KAKIZUKA, Akira, Kyoto-shi, Kyoto 606-8501 (JP); YOSHIDA, Tomoki, Kyoto-shi, Kyoto 606-8501 (JP); KABASHIMA, Kenji, Kyoto-shi, Kyoto 606-8501 (JP); SHIBUYA, Rintaro, Kyoto-shi, Kyoto 606-8501 (JP); SAKAMOTO, Mika, Kyoto-shi, Kyoto 606-8501 (JP)
(74) Representative: Hoffmann Eitle
(86) International application number: PCT/JP2022/019132
(87) International publication number: WO 2022/230949

(57) **Abstract**

The inventors have found that the compounds of formula (I) ameliorate skin tissue abnormalities. Accordingly, the disclosure provides a composition for ameliorating a skin tissue abnormality comprising a compound of formula (I) or an ester, oxide, pharmaceutically acceptable salt or solvate thereof.

## Description

### CROSS REFERENCE TO RELATED APPLICATION

This application claims the benefit of priority of Japanese Patent Application No. 2021-076617, the entire contents of which are incorporated herein by reference.

### BACKGROUND

### Technical Field:

The disclosure relates to a composition for ameliorating a skin tissue abnormality.

### Background Art:

The skin is an important organ that separates the body from the external environment and maintains homeostasis. The main functions of the skin include keeping water in the body, regulating the body temperature, protecting the body from external stimuli such as microorganisms, physical stimuli, and ultraviolet rays, detecting external information such as touch, warmth, and cold, discharging waste products from the body, secreting sebum and sweat, and storing subcutaneous fat. The skin plays such many roles and its abnormalities are associated with various diseases.

For example, itching is a symptom of many skin diseases. The diseases are aggravated when patients scratch their skin for itching. Dry skin causes reduced barrier function and makes foreign substances such as bacteria and irritants to enter the body easily. Dry skin also causes wrinkles and sagging, which are cosmetically undesirable.

Control of dry skin and itching is important in prevention and treatment of atopic dermatitis. Atopic dermatitis is a skin disease characterized by recurrent rashes with intense itching. Since dry skin aggravates atopic dermatitis, patients have to moisturize the skin routinely. Itching is one of the most distressing symptoms for patients with atopic dermatitis. Scratching caused by itching damages the skin and aggravates atopic dermatitis. Recent study reported that kallikrein 7 (KLK7), a serine protease, was highly expressed in the epidermis in an atopic dermatitis-like disease and that KLK7 promoted itching by a mechanism independent of inflammation (Non-Patent Literature 1).

Corticosteroids are used for treating atopic dermatitis. Known side effects of topical steroids include skin atrophy, telangiectasia, steroid acne, steroid flushing, hypertrichosis, and aggravation of bacterial, fungal, and viral infections. Known side effects of oral steroids include increased blood glucose levels, irritable gastric mucosa, and osteoporosis. Patients tend to dislike topical steroids, imagining the known side effects of oral steroids. An alternative to steroids is demanded for treating atopic dermatitis.

Certain 4-amino-naphthalene-1-sulfonic acid derivatives inhibit an ATPase activity of valosin-containing protein (VCP), a major ATPase in cells, and are expected to be effective for treating and/or preventing various diseases (Patent Literatures 1 to 8).

### Patent Literature

Patent Literature 1: WO2012/014994
Patent Literature 2: WO2012/043891
Patent Literature 3: WO2014/129495
Patent Literature 4: WO2015/129809
Patent Literature 5: WO2015/033981
Patent Literature 6: WO2019/131720
Patent Literature 7: WO2019/203176
Patent Literature 8: WO2020/027137

### Non-Patent Literature

Non-Patent Literature 1: Changxiong J., et al., Journal of Investigative Dermatology, volume 140, issue 6, Pages 1244-1252.e4

### SUMMARY

An object of the disclosure is to provide a new means for ameliorating a skin tissue abnormality.

The inventors have studied effects of VCP inhibitors on atopic dermatitis and found that the VCP inhibitors ameliorate skin tissue abnormalities.

Accordingly, an aspect of the disclosure provides a composition for ameliorating a skin tissue abnormality comprising a compound of formula (I): 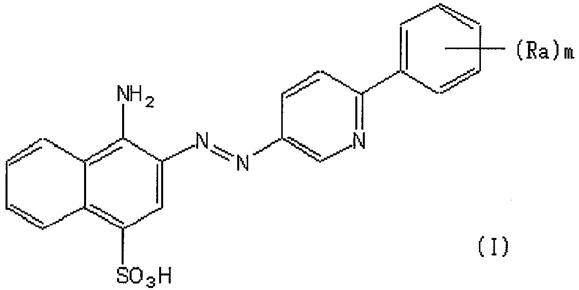 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt, or solvate thereof.

The disclosure provides the compound for ameliorating a skin tissue abnormality. The compound may be used in treatment and/or prevention of a skin disease or in cosmetic care.

### BRIEF DESCRIPTION OF DRAWINGS

Fig. 1 shows the appearance of the auricles of a model mouse of MC903-induced atopic dermatitis-like skin inflammation and a control mouse.
Fig. 2 shows the experimental schedule of Example 1 and the measured murine auricular thicknesses.
Fig. 3 shows the blood levels of immunoglobulins, IgE and IgG1, in mice.
Fig. 4 shows the representative HE staining images of murine auricular sections.
Fig. 5 shows the expression levels of messenger RNAs of Filaggrin (Flg) and Keratin 6a (Krt6a) in murine auricular sections.
Fig. 6 shows the levels of transepidermal water loss (TEWL) in mice.
Fig. 7 shows the experimental schedule of Example 4 and the measured murine auricular thicknesses.
Fig. 8 shows the measured scratching behaviors of mice.
Fig. 9 shows the expression levels of messenger RNA of KLK7 in murine auricular sections.
Fig. 10 shows the measured auricular thicknesses of model mice of imiquimod-induced psoriasis and control mice.
Fig. 11 shows the measured auricular thicknesses of model mice of sodium dodecyl sulfate (SDS)-induced irritant contact dermatitis and control mice.
Fig. 12 shows the appearance of the auricles and the measured auricular thicknesses when model mice of MC903-induced atopic dermatitis-like skin inflammation and control mice are treated with KUS187.

### DETAILED DESCRIPTION

When a numerical value is accompanied with the term "about", the value is intended to represent any value in the range of -10% of the value to +10% of the value. For example, "about 20" means "a value from 18 to 22". A range defined with values of the lower and upper limits covers all values from the lower limit to the upper limit, including the values of the both limits. When a range is accompanied with the term "about", the both limits are read as accompanied with the term. For example, "about 20 to 30" is read as "18 to 33".

Unless otherwise defined, the terms used herein are read as generally understood by those skilled in the technical fields such as organic chemistry, medical sciences, pharmaceutical sciences, molecular biology, and microbiology. Several terms used herein are defined as below. The definitions herein take precedence over the general understandings.

The term "alkyl" refers to a monovalent saturated aliphatic hydrocarbyl group having 1 to 10, preferably 1 to 6, carbon atoms. Examples of the alkyl groups include, but are not limited to, linear and branched hydrocarbyl groups, such as methyl, ethyl, n-propyl, isopropyl, n-butyl, isobutyl, sec-butyl, t-butyl, n-pentyl, and neopentyl.

The term "substituted" as a word qualifying a name of a group indicates that one or more hydrogen atoms of the group are, identically or differently, replaced with one or more designated substituents.

The term "alkylene" refers to a divalent saturated aliphatic hydrocarbyl group having 1 to 10, preferably 1 to 6, carbon atoms. The alkylene groups include branched and linear hydrocarbyl groups.

The term "alkoxy" refers to an -O-alkyl group in which the alkyl group is as defined herein. Examples of the alkoxy groups include, but are not limited to, methoxy, ethoxy, n-propoxy, isopropoxy, n-butoxy, t-butoxy, sec-butoxy, and n-pentoxy.

The term "aryl" refers to a monovalent aromatic carbocyclic group of 6 to 14 carbon atoms having a single ring (e.g., phenyl) or multiple condensed rings (e.g., naphthyl or anthryl). The aryl groups typically include phenyl and naphthyl.

The term "aryloxy" refers to an -O-aryl group in which the aryl group is as defined herein, including, e.g., phenoxy and naphthoxy.

The term "cyano" refers to the -CN group.

The term "carboxyl" or "carboxy" refers to the -COOH group or a salt thereof.

The term "carboxy ester" refers to a -C(O)O-alkyl group in which the alkyl group is as defined herein.

The term "halo" refers to a halogen, especially fluoro, chloro, bromo, or iodo.

The term "hydroxy" refers to the -OH group.

A substituent that is not explicitly defined herein is named by describing the name of the terminal functional group of the substituent first and sequentially describing the adjacent functional group(s) toward the binding point of the substituent, unless otherwise indicated. For example, the substituent "arylalkyloxycarbonyl" refers to (aryl)-(alkyl)-O-C(O)-.

Some compounds of formula (I) have enantiomers or diastereomers, depending on arrangements of their substituents. Some compounds of formula (I) may be provided as racemic mixtures or may be provided in stereoisomerically pure forms separated by a known method. Some compounds of formula (I) may be tautomers.

The term "ester" refers to an ester that does or does not hydrolyze in vivo, including an ester which breaks down readily in a human body to leave the parent compound or a salt thereof. Suitable esters include, for example, those derived from pharmaceutically acceptable aliphatic carboxylic acids, especially alkanoic, alkenoic, cycloalkanoic, and alkanedioic acids, in which each alkyl or alkenyl group has, for example, not more than six carbon atoms. Examples of the esters include formates, acetates, propionates, butyrates, acrylates, and ethylsuccinates.

The term "oxide" refers to an oxide in which a ring-nitrogen atom in a heteroaryl group is oxidized to form N-oxide.

The term "pharmaceutically acceptable salt" may indicate a salt of a compound of formula (I) with an inorganic or organic acid. Preferred salts include salts with inorganic acids, such as hydrochloric acid, hydrobromic acid, phosphoric acid, and sulfuric acid, and salts with organic carboxylic acids and sulfonic acids, such as acetic acid, trifluoroacetic acid, propionic acid, maleic acid, fumaric acid, malic acid, citric acid, tartaric acid, lactic acid, benzoic acid, methanesulfonic acid, ethanesulfonic acid, benzenesulfonic acid, toluenesulfonic acid, naphthalene sulfonic acid, and naphthalene disulfonic acid.

Pharmaceutically acceptable salts also include salts with conventional bases, such as alkali metal salts, e.g., a sodium salt and a potassium salt, alkaline earth metal salts, e.g., a calcium salt and a magnesium salt, ammonium salts derived from ammonia and organic amines, e.g., diethylamine, triethylamine, ethyldiisopropylamine, procaine, dibenzylamine, N-methylmorpholine, dihydroabiethylamine, methylpiperidine, L-arginine, creatine, choline, L-lysine, ethylenediamine, benzathine, ethanolamine, meglumine, and tromethamine, especially a sodium salt.

The term "solvate" means a compound of formula (I) which is coordinated with a solvent molecule to form a complex in a solid or liquid state. A suitable solvate is a hydrate.

The term "compound of formula (I)" as used herein is intended to include its esters, oxides, pharmaceutically acceptable salts, and solvates, as long as the context allows it.

Prodrugs of compounds of formula (I) also may be used. The term "prodrug" refers to a prodrug of a compound which may be used in contact with a human or animal tissue without an undue adverse effect such as toxicity, irritation, or allergic response within the scope of reasonable medical judgment, has a commensurate and reasonable benefit/risk ratio, and is effective in its intended use. The prodrug may be a compound which is rapidly transformed in vivo, for example by hydrolysis in blood, to yield a parent compound represented by the formula above. The prodrug may be a parent compound which is chemically modified and has enhanced biocompatibility. A general discussion is provided in T. Higuchi and V. Stella, Pro drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

In an embodiment, each Ra radical in formula (I) is independently selected from the group consisting of halo, hydroxy, alkyl, halo-substituted alkyl, alkoxy, and CHO.

In an embodiment, each Ra radical in formula (I) is independently selected from the group consisting of halo and alkyl.

In an embodiment, formula (I) has two Ra radicals which are halo and alkyl.

In an embodiment, each Ra radical in formula (I) is independently selected from the group consisting of alkyl, alkoxy, and CHO.

In an embodiment, formula (I) has three Ra radicals which are alkyl, alkoxy, and CHO.

In an embodiment, the compound of formula (I) is selected from the compounds listed in Table 1 below:

**[Table 1-1]**

| No. | Compound Name |
|---|---|
| 1 | 4-amino-3-(6-phenylpyridine-3-ylazo)naphthalene-1-sulfonic acid |
| 2 | 4-amino-3-(6-p-tolylpyridine-3-ylazo)naphthalene-1-sulfonic acid |
| 3 | 4-amino-3-(6-m-tolylpyridine-3-ylazo)naphthalene-1-sulfonic acid |
| 4 | 4-amino-3-(6-o-tolylpyridine-3-ylazo)naphthalene-1-sulfonic acid |
| 5 | 4-amino-3-(6-biphenyl-2-ylpyridine-3-ylazo)naphthalene-1-sulfonic acid |
| 6 | 3-[6-(2-acetylphenyl)pyridine-3-ylazo]-4-aminonaphthalene-1-sulfonic acid |
| 7 | 3-[6-(3-acetylphenyl)pyridine-3-ylazo]-4-aminonaphthalene-1-sulfonic acid |
| 8 | 3-[6-(4-acetylphenyl)pyridine-3-ylazo]-4-aminonaphthalenesulfonic acid |
| 9 | 4-amino-3-[6-(2,4-dichlorophenyl)pyridine-3-ylazo]naphthalene-1-sulfonic acid |
| 10 | 4-amino-3-[6-(2-trifluoromethylphenyl)pyridine-3-ylazo]naphthalene-1- sulfonic acid |
| 11 | 4-amino-3-[6-(4-trifluoromethylphenyl)pyridine-3-ylazo]naphthalene-1-sulfonic acid |
| 12 | 4-amino-3-[6-(2-chlorophenyl)pyridine-3-ylazo]naphthalene-1-sulfonic acid |
| 13 | 4-amino-3-[6-(3-chlorophenyl)pyridine-3-ylazo]naphthalene-1-sulfonic acid |
| 14 | 4-amino-3-[6-(4-chlorophenyl)pyridine-3-ylazo]naphthalene-1-sulfonic acid |
| 15 | 4-amino-3-[6-(2-methoxyphenyl)pyridine-3-ylazo]naphthalene-1-sulfonic acid |
| 16 | 4-amino-3-[6-(4-methoxyphenyl)pyridine-3-ylazo]naphthalene-1-sulfonic acid |
| 17 | 4-amino-3-[6-(2-isopropoxyphenyl)pyridine-3-ylazo]naphthalene-1-sulfonic acid |
| 18 | 4-amino-3-[6-(4-isopropoxyphenyl)pyridine-3-ylazo]naphthalene-1-sulfonic acid |
| 19 | 4-amino-3-[6-(2-phenoxyphenyl)pyridine-3-ylazo]naphthalene-1-sulfonic acid |
| 20 | 4-amino-3-[6-(3-methoxyphenyl)pyridine-3-ylazo]naphthalene-1-sulfonic acid |

**[Table 1-2]**

| | |
|---|---|
| 21 | 4-amino-3-[6-(2,3-dimethylphenyl)pyridine-3-ylazo]naphthalene-1-sulfonic acid |
| 22 | 4-amino-3-[6-(2,5-dimethylphenyl)pyridine-3-ylazo]naphthalene-1-sulfonic acid |
| 23 | 4-amino-3-[6-(3,5-dimethylphenyl)pyridine-3-ylazo]naphthalene-1-sulfonic acid |
| 24 | 4-amino-3-[6-(3-trifluoromethylphenyl)pyridine-3-ylazo]naphthalene-1- sulfonic acid |
| 25 | 4-{4-[5-(1-amino-4-sulfonaphthalene-2-ylazo)pyridine-2-yl]phenyl}-4-oxobutyric acid |
| 26 | 4-amino-3-(6-biphenyl-3-ylpyridine-3-ylazo)naphthalene-1-sulfonic acid |
| 27 | 4-amino-3-[6-(3-cyanophenyl)pyridine-3-ylazo]naphthalene-1-sulfonic acid |
| 28 | 4-amino-3-[6-(4-cyanophenyl)pyridine-3-ylazo]naphthalene-1-sulfonic acid |
| 29 | 4-amino-3-[6-(3,5-bistrifluoromethylphenyl)pyridine-3-ylazo]naphthalenesulfonic acid |
| 30 | 4-amino-3-[6-(4-benzoylphenyl)pyridine-3-ylazo]naphthalene-1-sulfonic acid |
| 31 | 4-amino-3-[6-(2-propoxyphenyl)pyridine-3-ylazo]naphthalene-1-sulfonic acid |
| 32 | 4-amino-3-[6-(4-fluoro-2-methylphenyl)pyridine-3-ylazo]naphthalene-1-sulfonic acid |
| 33 | 4-amino-3-[6-(5-fluoro-2-propoxyphenyl)pyridine-3-ylazo]naphthalene-1-sulfonic acid |
| 34 | 4-amino-3-[6-(2-fluoro-6-propoxyphenyl)pyridine-3-ylazo]naphthalene-1-sulfonic acid |
| 35 | 4-amino-3-[6-(4-fluoro-2-propoxyphenyl)pyridine-3-ylazo]naphthalene-1-sulfonic acid |
| 36 | 4-amino-3-[6-(5-fluoro-2-methylphenyl)pyridine-3-ylazo]naphthalene-1-sulfonic acid |
| 37 | 4-amino-3-[6-(2-fluoro-5-methylphenyl)pyridine-3-ylazo]naphthalene-1-sulfonic acid |
| 38 | 4-amino-3-[6-(2-butoxyphenyl)pyridine-3-ylazo]naphthalene-1-sulfonic acid |
| 39 | 4-amino-3-[6-(2-hexyloxyphenyl)pyridine-3-ylazo]naphthalene-1-sulfonic acid |
| 40 | 4-amino-3-[6-(4-butylphenyl)pyridine-3-ylazo]naphthalene-1-sulfonic acid |

**[Table 1-3]**

| | |
|---|---|
| 41 | 4-amino-3-[6-(2-hydroxyphenyl)pyridine-3-ylazo]naphthalene-1-sulfonic acid |
| 42 | 4-amino-3-{6-[2-(6-hydroxyhexyloxy)phenyl]pyridine-3-ylazo}naphthalene-1 - sulfonic acid |
| 43 | 4-{2-[5-(1-amino-4-sulfonaphthalene-2-ylazo)pyridine-2-yl]phenoxy}butyric acid |
| 44 | 4-amino-3-{6-[2-(3-hydroxypropoxy)phenyl]pyridine-3-ylazo}naphthalene-1- sulfonic acid |
| 45 | 4-amino-3-[6-(2-isobutoxyphenyl)pyridine-3-ylazo]naphthalene-1-sulfonic acid |
| 46 | 4-amino-3-[6-(5-chloro-2-hydroxyphenyl)pyridine-3-ylazo]naphthalene-1-sulfonic acid |
| 47 | 4-amino-3-[6-(4-methylbiphenyl-2-yl)pyridine-3-ylazo]naphthalene-1-sulfonic acid |
| 48 | 4-amino-3-[6-(4'-chloro-4-methylbiphenyl-2-yl)pyridine-3-ylazo]naphthalene-1-sulfonic acid |
| 49 | 4-amino-3-[6-(4,3',5'-trimethylbiphenyl-2-yl)pyridine-3-ylazo]naphthalene-1-sulfonic acid |
| 50 | 4-amino-3-[6-(3'-chloro-4-methylbiphenyl-2-yl)pyridine-3-ylazo]naphthalene-1-sulfonic acid |
| 51 | 4-amino-3-[6-(2,6-dimethylphenyl)pyridine-3-ylazo]naphthalene-1-sulfonic acid |
| 52 | 4-amino-3-[6-(3-formyl-2-isopropoxy-5-methylphenyl)pyridine-3-ylazo]naphthalene-1-sulfonic acid |
| 53 | 4-amino-3-[6-(3-formyl-2-butoxy-5-methylphenyl)pyridine-3-ylazo]naphthalene-1-sulfonic acid |

In an embodiment, 4-amino-3-[6-(4-fluoro-2-methylphenyl)pyridine-3-ylazo]naphthalene-1-sulfonic acid, which is represented by the following formula: 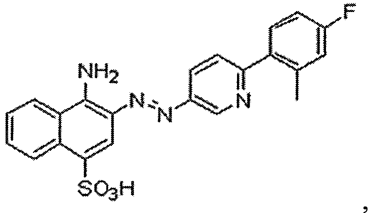 or an ester, oxide, pharmaceutically acceptable salt or solvate thereof, preferably a sodium salt, is used.

In an embodiment, 4-amino-3-[6-(3-formyl-2-butoxy-5-methylphenyl)pyridine-3-ylazo]naphthalene-1-sulfonic acid, which is represented by the following formula: 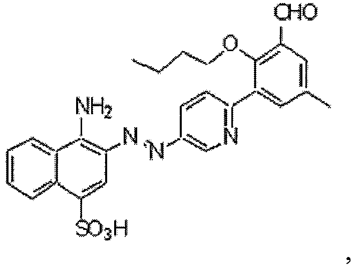 or an ester, oxide, pharmaceutically acceptable salt or solvate thereof, preferably a sodium salt, is used.

The characteristics of the compounds of formula (I), especially the compounds listed above, and the methods for synthesizing them are described in WO2012/014994 (Patent Literature 1) in detail.

As demonstrated by the Examples below, administering a compound of formula (I) can ameliorating a skin tissue abnormality. The term "skin tissue abnormality" as used herein includes symptoms of skin diseases and cosmetically undesirable conditions, e.g., erythema, swelling, dryness, and itching. The skin tissue may be at least one of epidermis, dermis, and subcutaneous tissues. The term "ameliorating a skin tissue abnormality" as used herein means reducing or eliminating a skin tissue abnormality, or preventing occurrence of a skin tissue abnormality, for a medical or non-medical (e.g., cosmetic) care. The term "medical care" as used herein means treatment or prevention of a disease performed under the judgment or direction of a physician. The term "non-medical care" as used herein means improvement of a living body other than medical care. In an embodiment, ameliorating a skin tissue abnormality is reducing or eliminating a skin tissue abnormality, preferably reducing or eliminating a skin tissue abnormality for medical care.

Examples of the skin diseases include allergic dermatitis (e.g., atopic dermatitis, allergic contact dermatitis, urticarial, insect bite, or actinic dermatitis), contact dermatitis (e.g., irritant contact dermatitis or photocontact dermatitis), cutaneous pruritus, asteatotic dermatitis, eczema, seborrheic dermatitis, erythema multiforme exudativum, and prurigo. In an embodiment, the skin disease is allergic dermatitis, contact dermatitis, cutaneous pruritus, or asteatotic dermatitis. In an embodiment, the skin disease is allergic dermatitis or contact dermatitis. In an embodiment, the skin disease is atopic dermatitis or irritant contact dermatitis. In an embodiment, the skin disease is allergic dermatitis. In an embodiment, the skin disease is atopic dermatitis.

The subjects to which a compound of formula (I) may be administered include animals, typically mammals (e.g., humans, mice, rats, hamsters, rabbits, cats, dogs, cows, sheep, or monkeys), especially humans.

A compound of formula (I) may be administered in any way through a generally known administration route, for example, by oral administration, parenteral administration, injection, or infusion. Parenteral administration may be systemic administration or topical administration, preferably topical administration. More specifically, topical administration may be application, spraying, or injection to a site of a skin abnormality. Parenteral administration also may be intravenous, intraarterial, intradermal, subcutaneous, transdermal, intramuscular, intraperitoneal or intranasal administration. In an embodiment, a compound of formula (I) is topically administered by application or spraying.

A composition comprising a compound of formula (I) may be administered. The composition may be solid, liquid, or any form in between (e.g., semi-solid). The composition may be any known dosage form and may be selected in view of the type of the skin tissue abnormality, the characteristics of the individual with the abnormality, the method of administration, and the dosage. For example, the composition may be a topical dosage form such as a spray, a lotion, a cream, a plaster, an ointment, a liquid, an emulsion, or a suspension. The composition may be a dosage form such as a tablet, a powder, a granule, a file granule, a capsule, a suppository, a solid injectable that dissolves upon use, a liquid injectable, an infusion, and an intravenous solution. A composition for cosmetic care may be a topical dosage form.

Such a dosage form can be manufactured by formulating an active ingredient by a conventional method. If necessary for the formulation, any of various pharmaceutically or cosmetically acceptable excipients may be added. Any excipient may be used in accordance with the employed dosage form. Examples of the excipients include buffering agents, surfactants, stabilizers, preservatives, fillers, diluents, additives, disintegrants, binders, coating agents, lubricants, lubricating agents, flavoring agents, sweeteners, solubilizers, coloring agents, dyes, and flavoring agents.

Dosage and number of doses of a compound of formula (I) may be appropriately set by those skilled in the art on the basis of factors such as the animal species, health condition, age, and weight of the subject, the administration route, and the employed dosage form so that an effective amount of the compound is administered to the subject. The effective amount in a given situation may be determined by routine experimentation. For example, a compound of formula (I) may be administered in the range of about 0.001 to about 1000 mg/kg body weight/day, about 0.01 to about 300 mg/kg body weight/day, or about 0.1 to about 100 mg/kg body weight/day. For example, an appropriate amount of a topical dosage form containing a compound of formula (I) at a concentration of about 0.01 to 10% by weight, about 0.05 to 5% by weight, or about 0.1 to 1% by weight may be applied or sprayed on the site of the skin abnormality.

A compound of formula (I) may be administered in a single dose or in multiple doses, or may be chronically administered. When administered in multiple doses, the compound may be administered, for example, once to several times a day, e.g., once, twice, or three times a day, daily or every few days, e.g., every one, two, three, or seven days. The duration of administration is not limited. For example, the administration may be continued until the skin abnormality is ameliorated. The administration may be interrupted temporarily.

A compound of formula (I) may be used alone or in combination with at least one further active ingredient, especially an active ingredient for ameliorating a skin tissue abnormality. When some ingredients are used in combination, a dosage form containing all the ingredients or a combination of dosage forms containing the ingredients separately may be employed. Multiple ingredients may be simultaneously administered or any ingredient may be administered at a later time point, as long as the ingredients are used for ameliorating a skin tissue abnormality. Two or more further active ingredients may be used in combination. For example, a composition containing at least one further active ingredient in addition to a compound of formula (I) may be used.

Examples of the active ingredients suitable for use in combination include nonsteroidal anti-inflammatory analgesics, steroids, antihistamines, antiallergic agents, immunosuppressants, antibiotics/antibacterial agents, antifungal agents, antiviral agents, anticancer agents, retinoids, sulfur preparations, zinc preparations, antiperspirants, talc, moisturizing preparations (e.g., urea preparations, preparations containing heparin-like substance), moisturizing ingredients (e.g., polyhydric alcohols such as glycerin, dipropylene glycol, 1,3-butylene glycol, and ethylene glycol, sugar alcohols such as sorbitol, and hyaluronic acid), UV absorbing agents (e.g., dimorpholinopyridazinone, octyl paramethoxysilicate, t-butyl methoxybenzoylmethane), UV scattering agents (e.g., fine-particle titanium dioxide, fine-particle zinc oxide), vitamins (e.g., L-ascorbic acid), plant extracts, and other cosmetic ingredients.

Administration of a compound of formula (I) may be combined with a non-drug medical or non-medical care, for example, laser therapy, skin grafting, regenerative medicine, surgical therapy, phototherapy, radiation therapy, cryotherapy, thermotherapy, local immunotherapy, iontophoresis, chemical peeling, or gene therapy.

A compound of formula (I) or a composition comprising it may be used for treating and/or preventing a skin disease.

The term "treating" or "treatment" as used herein means that in a subject suffering from a disease a cause of the disease is reduced or removed, progression of the disease is delayed or stopped, and/or a symptom of the disease is reduced or removed.

The term "preventing" or "prevention" as used herein means that in a subject, especially a subject that is susceptible to a disease but has not been affected with the disease yet, the disease onset is prevented or the possibility of the disease onset is decreased. Examples of the subjects that are susceptible to atopic dermatitis but have not been affected with it yet include subjects having risk factors of atopic dermatitis. Examples of the risk factors of atopic dermatitis include genetic predisposition, allergen exposure, stress, age, chemical stimuli, and physical stimuli.

The Examples below revealed that the compounds of formula (I) can moisturize abnormal skin and healthy skin. Accordingly, the compounds of formula (I) may be used for moisturizing skin for a medical or non-medical (e.g., cosmetic) care. For example, a composition comprising a compound of formula (I) may be used as a moisturizing composition for medical or cosmetic care.

An aspect of the disclosure provides a composition for ameliorating a skin tissue abnormality comprising a compound of formula (I).

An aspect of the disclosure provides a method for ameliorating a skin tissue abnormality comprising administering an effective amount of a compound of formula (I) to a subject in need thereof.

An aspect of the disclosure provides a compound of formula (I) for use in ameliorating a skin tissue abnormality.

An aspect of the disclosure provides use of a compound of formula (I) for ameliorating a skin tissue abnormality.

An aspect of the disclosure provides use of a compound of formula (I) in manufacturing a composition for ameliorating a skin tissue abnormality.

An aspect of the disclosure provides a composition for treating and/or preventing a skin disease comprising a compound of formula (I).

An aspect of the disclosure provides a method for treating and/or preventing a skin disease comprising administering an effective amount of a compound of formula (I) to a subject in need thereof.

An aspect of the disclosure provides a compound of formula (I) for use in treating and/or preventing a skin disease.

An aspect of the disclosure provides use of a compound of formula (I) for treating and/or preventing a skin disease.

An aspect of the disclosure provides use of a compound of formula (I) in manufacturing a composition for treating and/or preventing a skin disease.

An aspect of the disclosure provides a composition for moisturizing comprising a compound of formula (I).

An aspect of the disclosure provides a method for moisturizing skin comprising administering an effective amount of a compound of formula (I) to a subject in need thereof.

An aspect of the disclosure provides a compound of formula (I) for use in moisturizing.

An aspect of the disclosure provides use of a compound of formula (I) for moisturizing.

An aspect of the disclosure provides use of a compound of formula (I) in manufacturing a composition for moisturizing.

An aspect of the disclosure provides a composition for cosmetic care comprising a compound of formula (I).

An aspect of the disclosure provides a method for cosmetic care comprising administering an effective amount of a compound of formula (I) to a subject in need thereof.

An aspect of the disclosure provides a compound of formula (I) for use in cosmetic care.

An aspect of the disclosure provides use of a compound of formula (I) for cosmetic care.

An aspect of the disclosure provides use of a compound of formula (I) in manufacturing a composition for cosmetic care.

For example, the disclosure provides the following embodiments.
1. A composition for ameliorating a skin tissue abnormality, comprising a compound of formula (I): 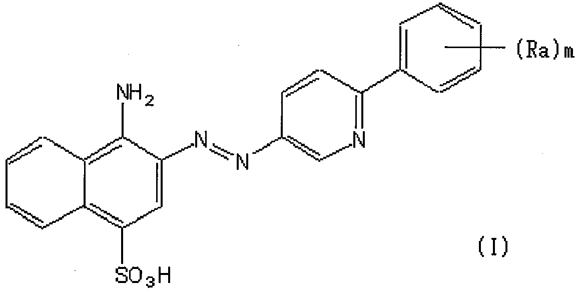 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt, or solvate thereof.
2. The composition according to item 1, wherein the skin tissue abnormality is swelling, dryness, or itching.
3. The composition according to item 1 or 2, wherein the skin tissue abnormality is dryness or itching.
4. The composition according to any one of items 1 to 3, wherein the skin tissue abnormality is dryness.
5. The composition according to any one of items 1 to 3, wherein the skin tissue abnormality is itching.
6. The composition according to any one of items 1 to 5, wherein the composition is a pharmaceutical composition.
7. The composition according to any one of items 1 to 6, wherein the skin tissue abnormality is a symptom of a skin disease.
8. The composition according to item 7, wherein the skin disease is allergic dermatitis, contact dermatitis, cutaneous pruritus, asteatotic dermatitis, eczema, seborrheic dermatitis, erythema multiforme exudativum, or prurigo.
9. The composition according to item 7 or 8, wherein the skin disease is allergic dermatitis, contact dermatitis, cutaneous pruritus, or asteatotic dermatitis.
10. The composition according to any one of items 7 to 9, wherein the skin disease is allergic dermatitis or contact dermatitis.
11. The composition according to any one of items 7 to 10, wherein the skin disease is atopic dermatitis or irritant contact dermatitis.
12. The composition according to any one of items 7 to 10, wherein the skin disease is allergic dermatitis.
13. The composition according to any one of items 7 to 12, wherein the skin disease is atopic dermatitis.
14. The composition according to any one of items 1 to 13, wherein the skin tissue abnormality is swelling, dryness, or itching in atopic dermatitis.
15. The composition according to any one of items 1 to 5, wherein the composition is a cosmetic composition.
16. A composition for treating and/or preventing a skin disease, comprising a compound of formula (I): 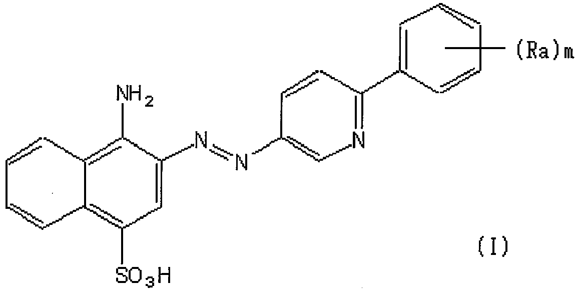 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt, or solvate thereof.
17. The composition according to item 16, wherein the skin disease is allergic dermatitis, contact dermatitis, cutaneous pruritus, asteatotic dermatitis, eczema, seborrheic dermatitis, erythema multiforme exudativum, or prurigo.
18. The composition according to item 16 or 17, wherein the skin disease is allergic dermatitis, contact dermatitis, cutaneous pruritus, or asteatotic dermatitis.
19. The composition according to any one of items 16 to 18, wherein the skin disease is allergic dermatitis or contact dermatitis.
20. The composition according to any one of items 16 to 19, wherein the skin disease is atopic dermatitis or irritant contact dermatitis.
21. The composition according to any one of items 16 to 19, wherein the skin disease is allergic dermatitis.
22. The composition according to any one of items 16 to 21, wherein the skin disease is atopic dermatitis.
23. A composition for moisturizing, comprising a compound of formula (I): 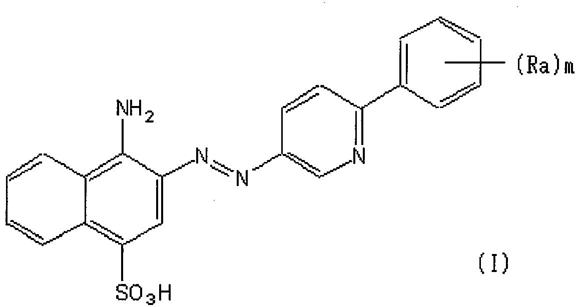 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt, or solvate thereof.
24. The composition according to item 23, wherein the composition is a pharmaceutical composition.
25. The composition according to item 23, wherein the composition is a cosmetic composition.
26. A composition for cosmetic care, comprising a compound of formula (I): 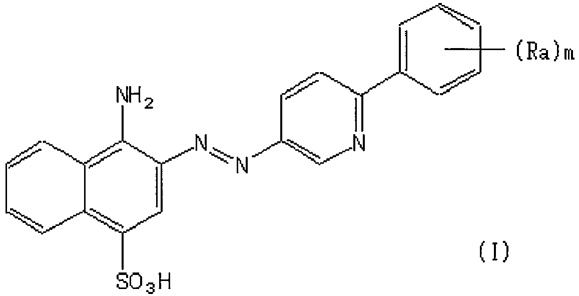 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt, or solvate thereof.
27. The composition according to any one of items 1 to 26, wherein each Ra radical is independently selected from the group consisting of halo, hydroxy, alkyl, halo-substituted alkyl, alkoxy, and CHO.
28. The composition according to any one of items 1 to 27, wherein each Ra radical is independently selected from the group consisting of halo and alkyl.
29. The composition according to any one of items 1 to 28, wherein formula (I) has two Ra radicals which are halo and alkyl.
30. The composition according to any one of items 1 to 27, wherein each Ra radical is independently selected from the group consisting of alkyl, alkoxy, and CHO.
31. The composition according to any one of items 1 to 27, wherein formula (I) has three Ra radicals which are alkyl, alkoxy, and CHO.
32. The composition according to any one of items 1 to 26, wherein the compound of formula (I) is selected from the compounds listed in Table 1.
33. The composition according to any one of items 1 to 27, wherein the compound of formula (I) is 4-amino-3-[6-(4-fluoro-2-methylphenyl)pyridine-3-ylazo]naphthalene-1-sulfonic acid or 4-amino-3-[6-(3-formyl-2-butoxy-5-methylphenyl)pyridine-3-ylazo]naphthalene-1-sulfonic acid.
34. The composition according to any one of items 1 to 27, wherein the compound of formula (I) is 4-amino-3-[6-(4-fluoro-2-methylphenyl)pyridine-3-ylazo]naphthalene-1-sulfonic acid.
35. The composition according to any one of items 1 to 27, comprising 4-amino-3-[6-(4-fluoro-2-methylphenyl)pyridine-3-ylazo]naphthalene-1-sulfonic acid sodium salt.
36. The composition according to any one of items 1 to 27, wherein the compound of formula (I) is 4-amino-3-[6-(3-formyl-2-butoxy-5-methylphenyl)pyridine-3-ylazo]naphthalene-1-sulfonic acid.
37. The composition according to any one of items 1 to 27, comprising 4-amino-3-[6-(3-formyl-2-butoxy-5-methylphenyl)pyridine-3-ylazo]naphthalene-1-sulfonic acid sodium salt.
38. The composition according to any one of items 1 to 37, wherein the composition is a topical dosage form.
39. The composition according to any one of items 1 to 38, wherein the composition is a spray, a lotion, a cream, a plaster, an ointment, a liquid, an emulsion, or a suspension.

The entire contents of the documents cited herein are incorporated herein by reference.

The following examples are non-limiting and provided only for describing the invention. The embodiments described above are non-limiting and may be modified without deviating from the scope of the invention as defined by the appended claims.

### EXAMPLES

In the test examples, the following compounds were used.
KUS121: 4-amino-3-[6-(4-fluoro-2-methylphenyl)pyridine-3-ylazo]naphthalene-1-sulfonic acid sodium salt
KUS187: 4-amino-3-[6-(3-formyl-2-butoxy-5-methylphenyl)pyridine-3-ylazo] naphthalene-1-sulfonic acid sodium salt.
KUS121 and KUS187 were prepared by the method disclosed in WO2012/014994.

### Example 1

The effect of KUS121 application on atopic dermatitis was evaluated. For modeling atopic dermatitis, model mice of atopic dermatitis-like skin inflammation induced by repeated applications of MC903, an active vitamin D3 analog, were used. Eight-week-old C57BL/6N female mice were used (purchased from SHIMIZU Laboratory Supplies Co., Ltd.). Under isoflurane anesthesia, the following substances were applied for 10 days. After 10 µL of 5 mM KUS121 (solvent: 90% EtOH) or 90% EtOH was applied to each side of the left auricle, 10 µL of 100 µM MC903 (solvent: 100% EtOH) was applied on each side. From the start of the substance application, KUS121 or 90% EtOH was applied daily and MC903 was applied every other day. The auricular thicknesses were measured immediately before the substance application using a constant pressured thickness measuring instrument (Tecloc). The thicknesses were measured immediately before the first substance application and then every other day for ten days. In addition, the effect of KUS121 alone was evaluated by the following substance applications. To each side of the left auricle 10 µL of 5 mM KUS121 was applied daily and to each side of the right auricle 10 µL of 90% EtOH was applied daily. The auricular thicknesses were measured immediately before the first substance application and then every other day for ten days. A blood sample was collected from a buccal vein on day 11 from the first substance application (14 hours after the substance application on day 10). The blood was allowed to stand at room temperature for at least 30 minutes, the serum was separated by centrifugation (1500 x g, 10 minutes), and the serum levels of immunoglobulins (IgE and IgG1) was measured using Elisa kit (Bethyl Laboratories).

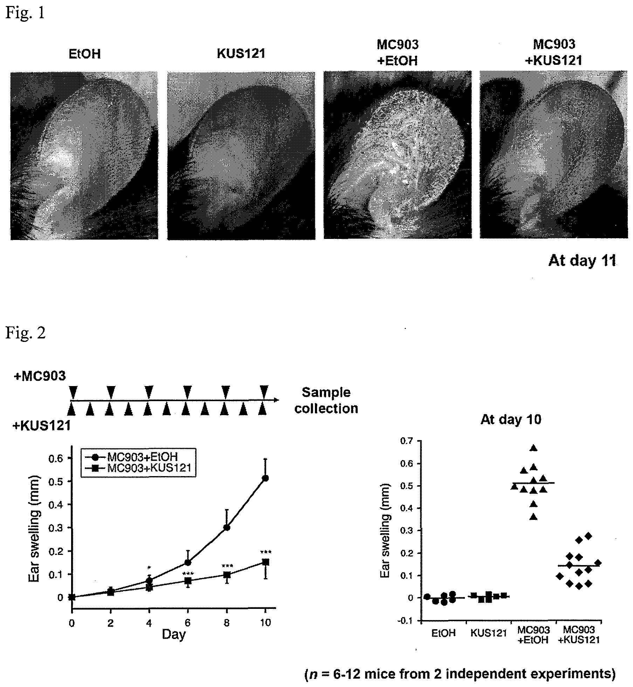

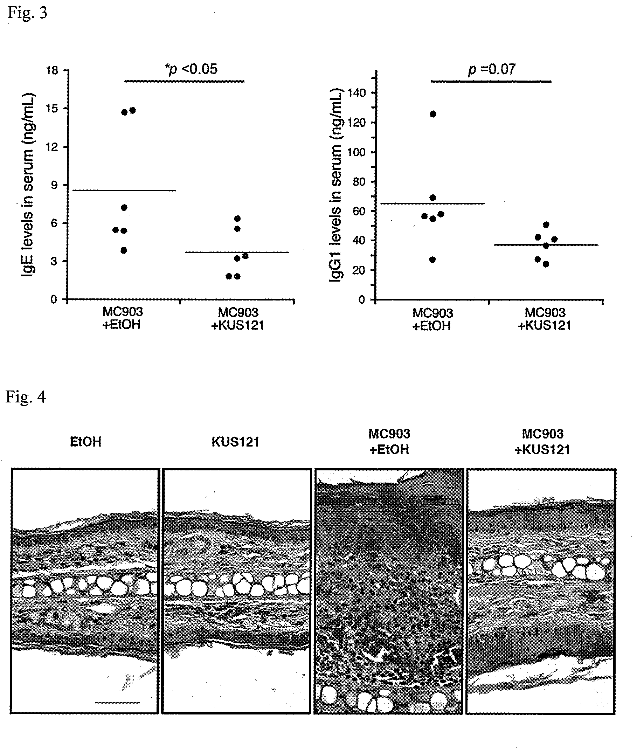

Fig. 1 shows the appearance of the left auricle on day 11 from the first substance application. Fig. 2 shows the experimental schedule and the measured auricular thicknesses. The mice applied with MC903 and treated with EtOH had skin redness, desquamation, and crust as well as increased auricular thickness. By contrast, the mice applied with MC903 and treated with KUS121 had less skin lesion and less increased auricular thickness. Fig. 3 shows the blood levels of IgE and IgG1, characteristic immunoglobulins in Th2 allergic reactions including atopic dermatitis. The blood levels of IgE and IgG1 were lower in the mice applied with MC903 and treated with KUS121 than in the mice applied with MC903 and treated with EtOH. The results suggest that KUS121 can suppress atopic dermatitis.

### Example 2

After the blood collection in Example 1, the mice were sacrificed by cervical dislocation and auricular sections were collected. Some of the auricular sections were paraffin-embedded and other sections were served for RNA extraction. The paraffin-embedded sections were observed by hematoxylin-eosin (HE) staining. RNA was extracted from the sections and cDNA was synthesized. The expression levels of various genes were analyzed by real-time PCR with SYBR Green using each cDNA as a template. Gapdh was used as an internal standard.

Fig. 4 shows the representative HE staining images. Epidermal thickening and infiltration of inflammatory cells such as eosinophils into the dermis were more frequently observed in the mice applied with MC903 and treated with EtOH than in the mice treated with EtOH alone or KUS121 alone. The lesions were suppressed more strongly in the mice applied with MC903 and treated with KUS121 than in the mice applied with MC903 and treated with EtOH.

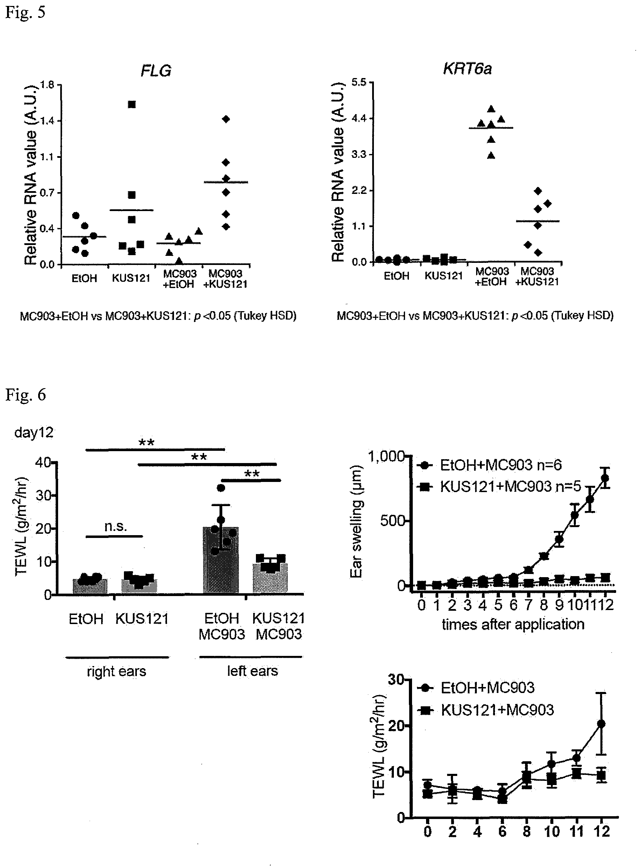

Fig. 5 shows the expression levels of each gene. Keratin 6a (Krt6a) is a characteristic keratin that is upregulated in skin injury, wound repair, and immune system initiation. Krt6a expression was higher in the mice applied with MC903 and treated with EtOH than in the mice treated with EtOH alone. Induction of Krt6a expression was weaker in the mice applied with MC903 and treated with KUS121. The results suggest that KUS121 can suppress skin injury associated with dermatitis. Filaggrin (Flg) is a natural moisturizing factor present in the upper epidermis and is known as a protein important for skin barrier function. Interestingly, Flg expression was equivalent in the mice applied with MC903 and treated with EtOH and the mice treated with EtOH alone, but was elevated in the mice applied with MC903 and treated with KUS121. The results suggest that KUS121 can moisturize skin by inducing filaggrin expression and maintaining skin barrier function.

### Example 3

The effect of KUS121 application on skin barrier function was evaluated by determining transepidermal water loss. Six- to eight-week-old C57BL6/N female mice (purchased from Oriental Bioservice, Inc) were used. The mice were anesthetized by subcutaneously injecting 150 µL of three types of mixed anesthesia (1 mg/mL medetomidine hydrochloride, 5 mg/mL mitazolam, 5 mg/mL butorphanol tartrate, solvent: sterile distilled water). Under anesthesia the following substances were applied for 12 days. For each mouse 10 µL of 5 mM KUS121 was applied to each side of the left auricle and 10 µL of 90% EtOH was applied to each side of the right auricle. Some mice were additionally applied with 10 µL of 100 µM MC903 on each side of both auricles every other day; KUS121 or 90% EtOH was applied at first and then MC903 was applied. The levels of transepidermal water loss was determined using APO SCAN AS-VT100 (Asahi Techno Lab. ltd.). The auricular thicknesses were measured immediately before the first substance application and daily for subsequent 12 days. The levels of transepidermal water loss were determined on days 0, 2, 4, 6, 8, 10, 11, and 12 immediately before the substance applications in each day.

Fig. 6 shows the results. The transepidermal water loss from auricular skin and the auricular thickness were more increased in the mice applied with MC903 and treated with EtOH than in the mice treated with EtOH alone. The increases were suppressed in the mice applied with MC903 and treated with KUS 121. The results suggest that KUS 121 is effective for maintaining skin barrier function.

### Example 4

The effect of KUS121 application on itching was evaluated. For modeling itching, model mice of atopic dermatitis-like skin inflammation induced by repeated applications of MC903 were used. Eight-week-old C57BL/6N female mice (purchased from SHIMIZU Laboratory Supplies Co., Ltd.) were used. Under isoflurane anesthesia, the following substances were applied for 15 days. 10 µL of 100 µM MC903 was applied on each side of both auricles every three days. To each side of the left auricle 10 µL of 5 mM KUS121 was applied daily and to each side of the right auricle 10 µL of 90% EtOH was applied daily. The application of KUS121 or 90% EtOH was started on day 6 from the first substance application; KUS121 or 90% EtOH was applied at first and then MC903 was applied. On day 12 from the start of the treatment, 1 to 2 hours after the substance application, five mice awakened from anesthesia were left in a shooting box (SHINANO manufacturing CO., LTD, W 25 x D 25 x H 30 cm) and their behavior was recorded from above using a video camera (Victor). Scratching behavior was evaluated by counting the total number of ear contacts by the front paws and determining the total scratching time by the hind paws within one hour from the start of the recording. The data of the five mice were summed and the total values were adopted as the results.

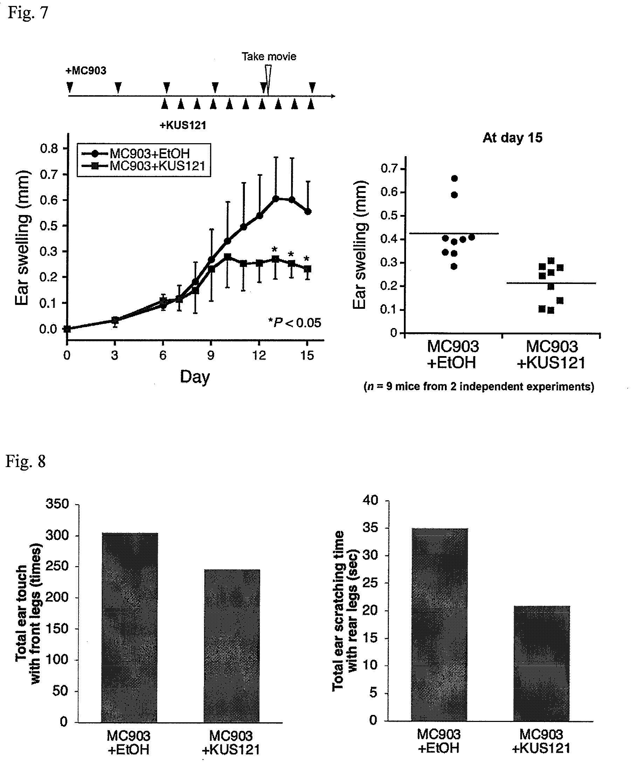

Fig. 7 shows the experimental schedule and the measured auricular thickness. Fig. 8 shows the measured results of the scratching behavior. Regarding the auricles applied with MC903 and KUS121, the scratching behavior was less in the mice applied with MC903 and treated with KUS121 than in the mice applied with MC903 and treated with EtOH. The increase of auricular thickness was suppressed when the KUS121 treatment was started after 6 days from the start of MC903 application. The results suggest that KUS 121 suppresses itching and the therapeutic effect can be achieved even KUS 121 is used after onset of atopic dermatitis.

### Example 5

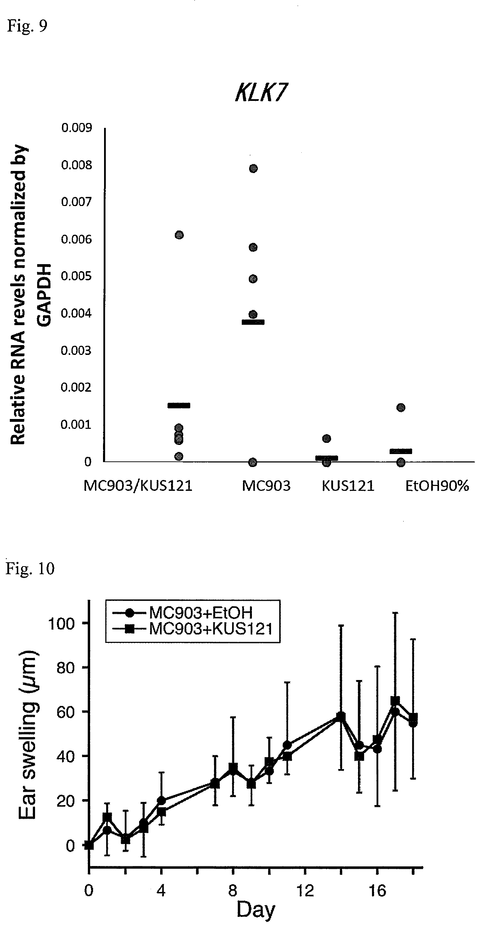

Mice were treated as in Example 1 and the expression level of KLK7 gene was analyzed as in Example 2. Fig. 9 shows the results. KLK7 is a serine protease that has been reported to promote itching by a mechanism independent of inflammation (Non-Patent Literature 1). KLK7 expression was increased in the mice applied with MC903 and treated with EtOH, whereas the increase was suppressed in the mice applied with MC903 and treated with KUS121. The results suggest that KUS121 suppresses KLK7 expression and thereby suppresses itching.

### Example 6

The effect of KUS121 application on psoriasis was evaluated. For modeling psoriasis, model mice of psoriasis induced by repeated applications of 5% imiquimod cream was used. Six- to eight-week-old C57BL6/N female mice (purchased from Oriental Bioservice, Inc) were used. The mice were anesthetized by subcutaneously injecting 150 µL of three types of mixed anesthesia. Under anesthesia the following substances were applied for 6 or 18 days. After 10 µL of 5 mM KUS121 or 90% EtOH was applied to each side of the left auricle, 5 mg of 5% imiquimod cream (MOCHIDA PHARMACEUTICAL CO., LTD.) was applied on each side. KUS121 or 90% EtOH was applied daily and imiquimod cream was applied on days 0, 2, 4, 7, 9, 11, 14, and 16. The auricular thicknesses were measured immediately before the substance applications.

Fig. 10 shows the results. No difference was found between the auricular thickness of the mice applied with 5% imiquimod cream and treated with EtOH and the auricular thickness of the mice applied with 5% imiquimod cream and treated with KUS121. The results suggest that KUS121 is not effective for psoriasis.

### Example 7

The effect of KUS121 application on irritant contact dermatitis was evaluated. For modeling irritant contact dermatitis, model mice of irritant contact dermatitis induced by sodium dodecyl sulfate (SDS) was used. Ten-week-old C57BL6/J male mice (purchased from Oriental Bioservice, Inc) were used. The mice were anesthetized by subcutaneously injecting 150 µL of three types of mixed anesthesia. Under anesthesia the following substances were applied for 4 days. After 10 µL of 5 mM KUS121 or 90% EtOH was applied to each side of the left auricle, 10 µL of 4% SDS was applied on each side. The substances were applied daily and the auricular thicknesses were measured immediately before the substance applications.

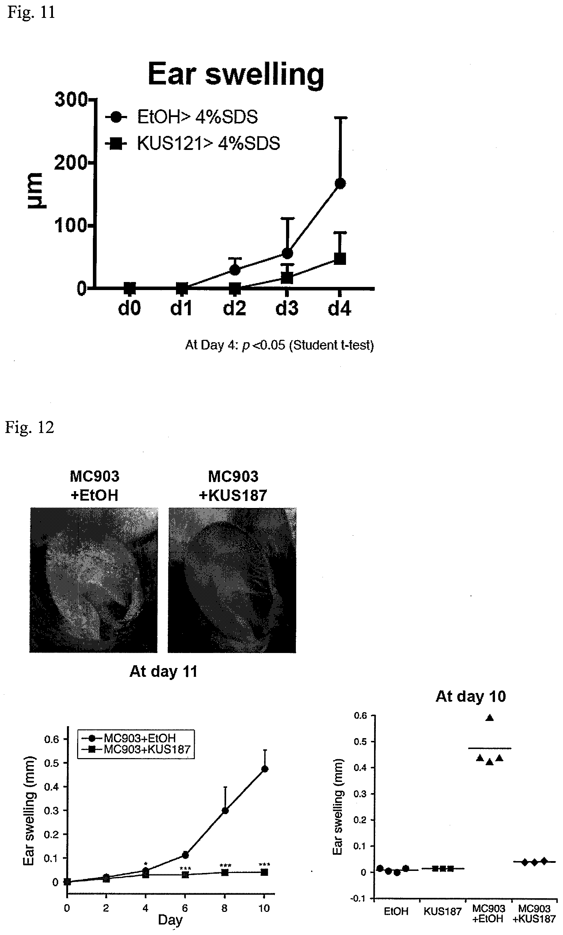

Fig. 11 shows the results. The auricular thickness was increased over time in the mice applied with SDS and treated with EtOH, whereas the increase was suppressed in the mice applied with SDS and treated with KUS121. The results suggest that KUS121 is effective for irritant contact dermatitis.

### Example 8

The experiments were performed as in Example 1, except for KUS187 was used in place of KUS121. The appearance of the auricle was observed and the auricular thicknesses were measured. Fig. 12 shows the results. The mice applied with MC903 and treated with EtOH had skin redness, desquamation, and crust as well as increased auricular thickness. By contrast, the mice applied with MC903 and treated with KUS 187 had less skin lesion and less increased auricular thickness. The results suggest that KUS187 can suppress atopic dermatitis.

### Industrial Applicability

The disclosure provides the method of ameliorating a skin tissue abnormality and may be used in the medical or cosmetic field. For example, the disclosure is expected to be used for treatment and/or prevention of a skin disease or cosmetic care.

## Claims

1. A composition for ameliorating a skin tissue abnormality, comprising a compound of formula (I): 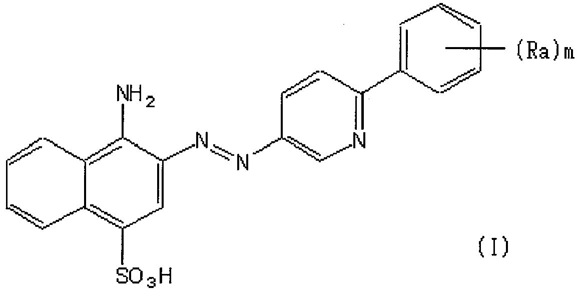 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt, or solvate thereof.

2. The composition according to claim 1, wherein each Ra radical is independently selected from the group consisting of halo, hydroxy, alkyl, halo-substituted alkyl, and alkoxy.

3. The pharmaceutical composition according to claim 1, wherein the compound of formula (I) is selected from the group consisting of
4-amino-3-(6-phenylpyridine-3-ylazo)naphthalene-1-sulfonic acid;
4-amino-3-(6-p-tolylpyridine-3-ylazo)naphthalene-1-sulfonic acid;
4-amino-3-(6-m-tolylpyridine-3-ylazo)naphthalene-1-sulfonic acid;
4-amino-3-(6-o-tolylpyridine-3-ylazo)naphthalene-1-sulfonic acid;
4-amino-3-(6-biphenyl-2-ylpyridine-3-ylazo)naphthalene-1-sulfonic acid;
3-[6-(2-acetylphenyl)pyridine-3-ylazo]-4-aminonaphthalene-1-sulfonic acid;
3 - [6-(3 -acetylphenyl)pyridine-3 -ylazo] -4-aminonaphthalene-1 -sulfonic acid;
3-[6-(4-acetylphenyl)pyridine-3-ylazo]-4-aminonaphthalenesulfonic acid;
4-amino-3-[6-(2,4-dichlorophenyl)pyridine-3-ylazo]naphthalene-1-sulfonic acid;
4-amino-3-[6-(2-trifluoromethylphenyl)pyridine-3-ylazo]naphthalene-1-sulfonic acid;
4-amino-3-[6-(4-trifluoromethylphenyl)pyridine-3-ylazo]naphthalene-1- sulfonic acid;
4-amino-3-[6-(2-chlorophenyl)pyridine-3-ylazo]naphthalene-1-sulfonic acid;
4-amino-3-[6-(3-chlorophenyl)pyridine-3-ylazo]naphthalene-1-sulfonic acid;
4-amino-3-[6-(4-chlorophenyl)pyridine-3-ylazo]naphthalene-1-sulfonic acid;
4-amino-3-[6-(2-methoxyphenyl)pyridine-3 -ylazo]naphthalene-1-sulfonic acid;
4-amino-3-[6-(4-methoxyphenyl)pyridine-3-ylazo]naphthalene-1-sulfonic acid;
4-amino-3-[6-(2-isopropoxyphenyl)pyridine-3-ylazo]naphthalene-1-sulfonic acid;
4-amino-3-[6-(4-isopropoxyphenyl)pyridine-3-ylazo]naphthalene-1-sulfonic acid;
4-amino-3-[6-(2-phenoxyphenyl)pyridine-3-ylazo]naphthalene-1-sulfonic acid;
4-amino-3-[6-(3-methoxyphenyl)pyridine-3-ylazo]naphthalene-1-sulfonic acid;
4-amino-3-[6-(2,3-dimethylphenyl)pyridine-3-ylazo]naphthalene-1-sulfonic acid;
4-amino-3-[6-(2,5-dimethylphenyl)pyridine-3-ylazo]naphthalene-1-sulfonic acid;
4-amino-3-[6-(3,5-dimethylphenyl)pyridine-3-ylazo]naphthalene-1-sulfonic acid;
4-amino-3-[6-(3-trifluoromethylphenyl)pyridine-3-ylazo]naphthalene-1-sulfonic acid;
4-{4-[5-(1-amino-4-sulfonaphthalene-2-ylazo)pyridine-2-yl]phenyl}-4-oxobutyric acid;
4-amino-3-(6-biphenyl-3-ylpyridine-3-ylazo)naphthalene-1-sulfonic acid;
4-amino-3-[6-(3-cyanophenyl)pyridine-3-ylazo]naphthalene-1-sulfonic acid;
4-amino-3-[6-(4-cyanophenyl)pyridine-3-ylazo]naphthalene-1-sulfonic acid;
4-amino-3-[6-(3,5-bistrifluoromethylphenyl)pyridine-3-ylazo]naphthalenesulfonic acid;
4-amino-3-[6-(4-benzoylphenyl)pyridine-3-ylazo]naphthalene-1-sulfonic acid;
4-amino-3-[6-(2-propoxyphenyl)pyridine-3-ylazo]naphthalene-1-sulfonic acid;
4-amino-3-[6-(4-fluoro-2-methylphenyl)pyridine-3-ylazo]naphthalene-1-sulfonic acid;
4-amino-3-[6-(5-fluoro-2-propoxyphenyl)pyridine-3-ylazo]naphthalene-1-sulfonic acid;
4-amino-3-[6-(2-fluoro-6-propoxyphenyl)pyridine-3-ylazo]naphthalene-1-sulfonic acid;
4-amino-3-[6-(4-fluoro-2-propoxyphenyl)pyridine-3-ylazo]naphthalene-1-sulfonic acid;
4-amino-3-[6-(5-fluoro-2-methylphenyl)pyridine-3-ylazo]naphthalene-1-sulfonic acid;
4-amino-3-[6-(2-fluoro-5-methylphenyl)pyridine-3-ylazo]naphthalene-1-sulfonic acid;
4-amino-3-[6-(2-butoxyphenyl)pyridine-3-ylazo]naphthalene-1-sulfonic acid;
4-amino-3-[6-(2-hexyloxyphenyl)pyridine-3-ylazo]naphthalene-1-sulfonic acid;
4-amino-3-[6-(4-butylphenyl)pyridine-3-ylazo]naphthalene-1-sulfonic acid;
4-amino-3-[6-(2-hydroxyphenyl)pyridine-3-ylazo]naphthalene-1-sulfonic acid;
4-amino-3-{6-[2-(6-hydroxyhexyloxy)phenyl]pyridine-3-ylazo}naphthalene-1-sulfonic acid;
4-{2-[5-(1-amino-4-sulfonaphthalene-2-ylazo)pyridine-2-yl]phenoxy} butyric acid;
4-amino-3-{6-[2-(3-hydroxypropoxy)phenyl]pyridine-3-ylazo}naphthalene-1-sulfonic acid;
4-amino-3-[6-(2-isobutoxyphenyl)pyridine-3-ylazo]naphthalene-1-sulfonic acid;
4-amino-3-[6-(5-chloro-2-hydroxyphenyl)pyridine-3-ylazo]naphthalene-1-sulfonic acid;
4-amino-3-[6-(4-methylbiphenyl-2-yl)pyridine-3 -ylazo]naphthalene-1 -sulfonic acid;
4-amino-3-[6-(4'-chloro-4-methylbiphenyl-2-yl)pyridine-3-ylazo]naphthalene-1-sulfonic acid;
4-amino-3-[6-(4,3',5'-trimethylbiphenyl-2-yl)pyridine-3-ylazo]naphthalene-1-sulfonic acid;
4-amino-3-[6-(3'-chloro-4-methylbiphenyl-2-yl)pyridine-3-ylazo]naphthalene-1-sulfonic acid;
4-amino-3-[6-(2,6-dimethylphenyl)pyridine-3-ylazo]naphthalene-1-sulfonic acid;
4-amino-3-[6-(3-formyl-2-isopropoxy-5-methylphenyl)pyridine-3-ylazo]naphthalene-1-sulfonic acid; and
4-amino-3-[6-(3-formyl-2-butoxy-5-methylphenyl)pyridine-3-ylazo]naphthalene-1-sulfonic acid.

4. The composition according to any one of claims 1 to 3, wherein the compound of formula (I) is 4-amino-3-[6-(4-fluoro-2-methylphenyl)pyridine-3-ylazo]naphthalene-1-sulfonic acid or 4-amino-3-[6-(3-formyl-2-butoxy-5-methylphenyl)pyridine-3 -ylazo]naphthalene-1-sulfonic acid.

5. The composition according to any one of claims 1 to 4, wherein the skin tissue abnormality is swelling, dryness, or itching.

6. The composition according to any one of claims 1 to 5, wherein the composition is a pharmaceutical composition.

7. The composition according to any one of claims 1 to 6, wherein the skin tissue abnormality is a symptom of atopic dermatitis or irritant contact dermatitis.

8. The composition according to any one of claims 1 to 5, wherein the composition is a cosmetic composition.

9. The composition according to any one of claims 1 to 8, wherein the composition is a topical dosage form.

10. The composition according to any one of claims 1 to 9, wherein the composition is a spray, a lotion, a cream, a plaster, an ointment, a liquid, an emulsion, or a suspension.
